# EUROPEAN PATENT APPLICATION

(11) **EP 4 806 461 A1**
(43) Date of publication of application: **16.09.2026**
(21) Application number: 25197247.7
(22) Date of filing: 21.08.2025
(51) Int. Cl.: A61K 39/02, A61P 31/04, G01N 33/58

(54) **ROUGH ATTENUATED BRUCELLA STRAIN, USE, VACCINE, ANTIGEN, AND KIT THEREOF**

(30) Priority: 10.03.2025 CN 202510273083
(71) Applicant: Institute of Animal Sciences of Chinese Academy of Agricultural Sciences, Beijing 100193 (CN)
(72) Inventor: DING, Jiabo, Beijing 100193 (CN); JIANG, Hui, Beijing 100193 (CN); FENG, Yu, Beijing 100193 (CN); FAN, Xuezheng, Beijing 100193 (CN); LI, Linjiao, Beijing 100193 (CN); SHEN, Qingchun, Beijing 100193 (CN)
(74) Representative: Berggren Oy

(57) **Abstract**

The present invention relates to the field of veterinary biological products. Particularly, the invention relates to a rough attenuated strain RB39 of *Brucella,* use, vaccine, antigen, and kit thereof. The rough attenuated strain RB39 of *Brucella* according to the present invention is able to be used to prepare a vaccine with high safety. The vaccine may be injected into pregnant animals to provide effective immunity. Moreover, the vaccine is applicable for immunization of multiple animal species. Additionally, based on the rough attenuated strain RB39 of *Brucella* of the present invention, an antigen for differentiating rough *Brucella* antibodies may be prepared, effectively distinguishing between vaccination and natural infection, thus providing a scientific basis for brucellosis control.

## Description

### TECHNICAL FIELD

The present invention relates to the field of veterinary biological products, particularly to a rough attenuated *Brucella* strain, use, vaccine, antigen, and kit thereof.

### BACKGROUND

Brucellosis is a zoonotic disease caused by *Brucella* and characterized by abortion and fever, posing severe threats to human and animal health. Human infections primarily result from contact with infected animals or contaminated animal-derived products. Therefore, vaccine immunization against animal brucellosis in high-prevalence regions is a globally recognized effective measure for brucellosis control. Current veterinary brucellosis vaccines are mostly prepared from smooth attenuated *Brucella* strains, whose primary drawback lies in their relatively high virulence preventing injection into pregnant animals; vaccine-induced smooth antibodies are unable to be differentiated from antibodies produced by naturally infected smooth virulent strains, leading to mistaken identification of vaccinated animals as infection sources and consequent culling; or positive animals in herds may be misidentified as vaccinated animals rather than eliminated, perpetuating infection reservoirs and preventing disease eradication. Common existing methods employ genetically engineered marker vaccines to differentiate between natural infection and vaccination, but the methods entail high costs and operational complexity. These limitations restrict the adoption and widespread use of brucellosis vaccines.

Rough attenuated *Brucella* strains offer higher safety compared to smooth attenuated *Brucella* strains. Following injection, the strains stimulate production of rough antibodies that are effectively distinguishable from smooth antibodies generated by naturally infected smooth virulent strains, representing a primary direction for novel brucellosis vaccine development. Currently, RB51 is the only globally approved and widely used rough vaccine strain. Developed in the 1990s by U.S. scientists through rifampicin-induced mutagenesis, this vaccine demonstrates good immunogenicity and is deployed in multiple countries across the Americas. However, its safety remains controversial because rifampicin is an effective antibiotic for human brucellosis treatment and the vaccine was derived from virulent strain induction; although the virulence thereof has been attenuated, administration to pregnant animals still causes adverse effects including abortion. Furthermore, existing rough vaccines are applicable only to specific animal species, failing to cover all susceptible animals and thus limiting their broad application.

### SUMMARY

The technical problem to be solved by the present invention is to provide a rough attenuated *Brucella* strain that has high safety without causing abortion or other adverse effects, is applicable to multiple animal species, stimulates production of specific antibodies, enables differentiation between natural infection and vaccination, thereby effectively controlling brucellosis transmission and expanding vaccine applicability.

The present invention further aims to provide use of the rough attenuated *Brucella* strain in preparing a brucellosis vaccine or an antigen for detection of *Brucella* antibodies.

The present invention further aims to provide a brucellosis vaccine with high safety that is injectable into pregnant animals and applicable to multiple animal species.

The present invention further aims to provide an antigen with high specificity capable of effectively differentiating between natural infection and vaccination.

The present invention further aims to provide a kit capable of effectively differentiating between natural infection and vaccination.

To address the above technical problems, as a first aspect of the present invention, the invention provides a rough attenuated strain RB39 of *Brucella abortus* (Rough type, *B. abortus*), deposited on February 17, 2025 at the China General Microbiological Culture Collection Center (CGMCC) under accession number CGMCC 46377, biologically classified as a rough attenuated *Brucella* strain, with the address of the China General Microbiological Culture Collection Center being at the Institute of Microbiology, Chinese Academy of Sciences, No. 3, Yard. 1, Beichen West Road, Chaoyang District, Beijing, China.

Specifically, the strain RB39 is a stable rough attenuated *Brucella* strain obtained by subjecting the smooth attenuated *Brucella* strain A19 (CVCC 70202) to 39 serial passages in TSB medium at pH 5.5, followed by 65 serial passages in TSB medium at pH 4.5, and finally 35 serial passages in mice. The colonies thereof are identified as 100% rough phenotype by crystal violet staining assay and as *Brucella abortus* by AMOS-PCR assay. The strain RB39 not only exhibits high safety but also significant immunogenicity, is applicable to multiple animal species including cattle, sheep, and swine, effectively stimulates production of specific antibodies, allows differentiation between natural infection and vaccination status, and significantly improves brucellosis control efficacy.

As a second aspect of the present invention, the invention discloses use of the aforementioned rough attenuated strain RB39 of *Brucella* in preparing a brucellosis vaccine. The vaccine possesses high safety and strong immunogenicity, and does not cause abortion or other adverse effects when administered to pregnant animals, ensuring maternal and fetal safety. Moreover, the vaccine is applicable to multiple animal species (including but not limited to mouse, guinea pig, cattle, goat, sheep, and swine), significantly enhancing protective efficacy and effectively controlling brucellosis transmission.

As a third aspect of the present invention, the invention provides a brucellosis vaccine including the aforementioned rough attenuated strain RB39 of *Brucella.* Specifically, the brucellosis vaccine is prepared by inactivating the rough attenuated strain RB39 of *Brucella* followed by adding pharmaceutical excipients. The pharmaceutical excipients for the brucellosis vaccine include, but are not limited to, adjuvants, stabilizers, preservatives, diluents, and antioxidants.

Preferably, in some embodiments, the vaccine includes a lyoprotectant including, but is not limited to, PEG, gelatin, lactose, and glycine. The lyoprotectant prevents damage to strain viability during lyophilization, ensures vaccine stability, and further enhances practical application efficacy of the vaccine.

Specifically, when applying the brucellosis vaccine of the present invention, subcutaneous injection of pregnant animals at a dose of one cattle dose (8.0 × 10¹⁰ CFU to 1.6 × 10¹¹ CFU) for cattle or 1/4 sheep dose (2.0 × 10¹⁰ CFU to 4.0 × 10¹⁰ CFU) for sheep causes no abortion.

As a fourth aspect of the present invention, the invention further provides use of the aforementioned rough attenuated strain RB39 of *Brucella* in preparing an antigen for detection of *Brucella* antibodies produced by an animal immunized with the aforementioned brucellosis vaccine; the detection allows differentiation between vaccination and natural infection, improves diagnostic accuracy, facilitates precise brucellosis control, and ensures healthy development of animal husbandry.

Specifically, the detection is serological detection characterized by simplicity and high efficiency. More specifically, the detection may employ, but is not limited to, the Rose Bengal Test (RBT) method, the Serum Agglutination Test (SAT) method, the Complement Fixation Test (CFT) method, or the Enzyme-Linked Immunosorbent Assay (ELISA) method. Preferably, the detection employs the RBT method featuring simple operation, rapid completion under field conditions, and reliable results. Suitable for large-scale screening, it enables rapid and effective differentiation between natural infection and vaccination, providing a scientific basis for formulating brucellosis control strategies.

As a fifth aspect of the present invention, the invention further provides an antigen for detection of *Brucella* antibodies produced by an animal immunized with the aforementioned brucellosis vaccine, the antigen being obtained by culturing and inactivating the aforementioned rough attenuated strain RB39 of *Brucella.*

Specifically, additional processing of the antigen is required depending on the detection method. For example: for the RBT method, staining is performed on the inactivated antigen; for the CFT method, operations including disruption, LPS extraction, and purification are applied to the antigen; for the ELISA method, operations including disruption, LPS extraction, and coating treatment are applied to the antigen. It should be noted that a person skilled in the art may optimize the antigen according to specific detection methods to ensure accuracy and reliability of detection results.

Preferably, in some embodiments, the antigen is for detection of *Brucella* antibodies by the RBT method, and a preparation method thereof includes:
preparing a bacterial suspension from the aforementioned rough attenuated strain RB39 of *Brucella* using physiological saline, followed by inactivation;
adding Rose Bengal dye to the bacterial suspension after inactivation at a volume ratio of 0.5:100 for staining to obtain stained bacterial cells;
resuspending the stained bacterial cells in Tris buffer to a predetermined concentration.

More preferably, the preparation method includes: preparing a concentrated bacterial suspension from the aforementioned rough attenuated strain RB39 of *Brucella* using physiological saline, inactivating the bacterial suspension, adding Rose Bengal dye at 4% concentration at a volume ratio of 0.5% for staining, resuspending the stained bacterial cells in Tris buffer at a predetermined ratio, further diluting with Tris buffer to 1:3, 1:4, 1:5, and 1:6 fold dilutions, mixing 30 µL of each diluted antigen with 30 µL each of rough *Brucella* RB39 strain positive serum at 10 IU/mL and rough *Brucella* RB39 strain positive serum at 5 IU/mL, and observing the reaction results within 4 minutes; where the antigen dilution that causes the rough *Brucella* RB39 strain positive serum at 10 IU/mL to show a "+" reaction and causes the rough Brucella RB39 strain positive serum at 5 IU/mL to show a "-" reaction is determined as the antigen use concentration.

As a sixth aspect of the present invention, the invention further provides a kit for detection of *Brucella* antibodies, including the aforementioned antigen.

Implementation of the present invention achieves the following beneficial effects:
1. The present invention develops a rough attenuated strain RB39 of *Brucella* and vaccine thereof that is able to be subcutaneously injected into pregnant animals without causing abortion, overcoming the limitation of current brucellosis vaccines which are unable to be administered to pregnant animals; moreover, the vaccine is applicable for immunization of multiple animal species. Specifically, when immunizing mice, guinea pigs, cattle, goats, and sheep at specified doses, the vaccine provides no less than 70% protection rate.
2. The present invention develops an antigen for differential diagnosis between RB39 strain vaccination and natural infection. Based on this antigen, serological testing methods can rapidly differentiate antibodies induced by vaccination from those produced by natural infection, resolving the technical challenge of distinguishing vaccinated from naturally infected animals in current brucellosis vaccines, and providing a scientific basis for brucellosis control.

### BRIEF DESCRIPTION OF THE DRAWINGS

FIG. 1 is an electrophoretogram of AMOS-PCR for the rough *Brucella* RB39 strain;
FIG. 2 is a circular diagram of the genome of the rough *Brucella* RB39 strain;
FIG. 3 is an antibody level variation diagram in cattle after immunization with the rough *Brucella* RB39 strain;
FIG. 4 is an antibody level variation diagram in sheep after immunization with the rough *Brucella* RB39 strain.

### DETAILED DESCRIPTION

To make the objectives, technical solutions, and advantages of the present invention clearer, the invention is described in further detail below. Unless otherwise specified, reagents used in the examples are commercially available.

### Example: Induction and Adaptation of the RB39 Strain

1.1 Induction under acidic condition at pH 5.5: A smooth *Brucella* strain A19 (CVCC 70202, purchased from commercial *Brucella* live vaccine A19) was continuously passaged on Tryptic Soy Broth (TSB) medium at pH 5.5. Every three passages, a limited dilution method was used to spread onto Trypticase Soy Agar (TSA) plates, followed by incubation at 37°C for 3 days. After single colonies appeared, colonies on the plates were stained with crystal violet. The colony with the most pronounced crystal violet staining (rough colonies stained while smooth colonies remained unstained) was selected for continued passage. If no crystal violet stain-positive colony appeared, colonies were randomly picked. After 39 passages by this method, the result showed that the positive rate of crystal violet-stained rough colonies exceeded 20%.

1.2 Induction under acidic condition at pH 4.5: The rough colony picked in 1.1 was further passaged on TSB medium at pH 4.5. Every five passages, a limited dilution method was used to spread onto TSA plates, followed by incubation at 37°C for 3 days. After single colonies appeared, colonies on the plates were stained with crystal violet. The colony with the most pronounced crystal violet staining was selected for continued passage. After 65 consecutive passages, the result showed that the positive rate of crystal violet-stained rough colonies exceeded 90%.

1.3 Passage and adaptation in mice: The rough colony picked in 1.2 was subcutaneously injected into the groin of one BALB/c mouse at a dose of 1.0×10⁸ CFU per mouse. Seven days later, the mouse was sacrificed, and spleen tissue was homogenized. A limited dilution method was used to spread onto TSA plates, followed by incubation at 37°C for 3 days. After single colonies appeared, colonies on the plates were stained with crystal violet. The colony with the most pronounced crystal violet staining was selected for continued passage. After 25 consecutive passages, the result showed that the positive rate of crystal violet-stained rough colonies reached 100%. Subsequently, 10 additional passages were performed in mice (totaling 139 passages). The results demonstrated that the positive rate of isolated rough colonies remained 100% for 10 consecutive passages. The genetically stable rough *Brucella* strain obtained was designated as the RB39 strain.

### Example 2 Identification of the Rough Brucella RB39 Strain

2.1 Morphological and Biochemical Characteristics: The strain was a Gram-negative coccobacillus, lacking spores and capsules. Gram staining result was negative. Hydrogen sulfide production test was positive.

2.2 Cultural Characteristics: The strain grew well on Trypticase Soy Agar (TSA) medium. After streaking on the aforementioned agar plates and incubating at 37°C for 3 days, 100% of the colonies belonged to the rough type. The strain grew uniformly, turbidly, and opaquely in liquid media such as Tryptic Soy Broth (TSB).

2.3 Variation Check: The strain conformed to the characteristics of rough-type colonies. Crystal violet staining assay of colonies showed 100% of colonies were stained. Heat agglutination test resulted in precipitation. Acriflavine agglutination test resulted in agglutination.

2.4 Serological Characteristics: An antigen prepared from RB39 culture did not form agglutination with smooth *Brucella*-positive serum, but formed agglutination with rough *Brucella-*positive serum.

2.5 PCR Identification: Identification was performed using the AMOS-PCR method. The following 4 primers were synthesized (see Table 1).

**Table 1 AMOS-PCR Primers**

| Primer Name | Sequence (5'-3') | Amplification Product Size (bp) |
|---|---|---|
| Fₛᵤᵢₛ | GCGCGGTTTTCTGAAGGTTCAGG | 285 |
| Fₘₑₗᵢₜₑₙₛᵢₛ | AAATCGCGTCCTTGCTGGTCTG | 731 |
| F_{abortus} | GACGAACGGAATTTTTCCAATCCC | 498 |
| R (Universal) | TGCCGATCACTTAAGGGCCTTCAT | |

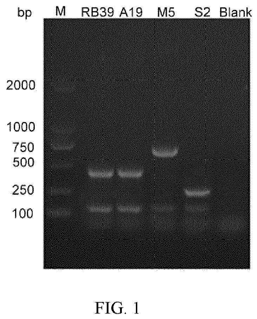

Genomic DNA of RB39 was extracted using a commercial bacterial genomic DNA extraction kit and used as the template. In a 20 µL reaction system, the following components were added: 10 µL of 2× PCR mix, 1 µL of each of the four primers, 1 µL of template DNA, and 5 µL of DEPC-treated water. The mixture was thoroughly mixed for PCR amplification. Nucleic acid control groups were simultaneously set up: *Brucella abortus* strain A19 (corresponding to primer F_{abortus}), *Brucella melitensis* strain M5 (CVCC 18, purchased from commercial Brucella live vaccine M5, corresponding to primer Fₘₑₗᵢₜₑₙₛᵢₛ), *Brucella suis* strain S2 (CVCC 70502, purchased from commercial *Brucella* live vaccine S2, corresponding to primer Fₛᵤᵢₛ), and a blank control group without nucleic acid addition. The PCR reaction program was: initial denaturation at 95°C for 5 min; followed by 35 cycles of denaturation at 95°C for 30 s, annealing at 54°C for 30 s, and extension at 72°C for 1 min; and a final extension at 72°C for 10 min. The amplification products were identified by electrophoresis on a 1.5% agarose gel. RB39 amplified two specific PCR bands, with sizes of 178 bp and 498 bp, identifying it as *Brucella abortus,* as shown in FIG. 1.

### Example 3 Whole Genome Sequencing of RB39 Strain

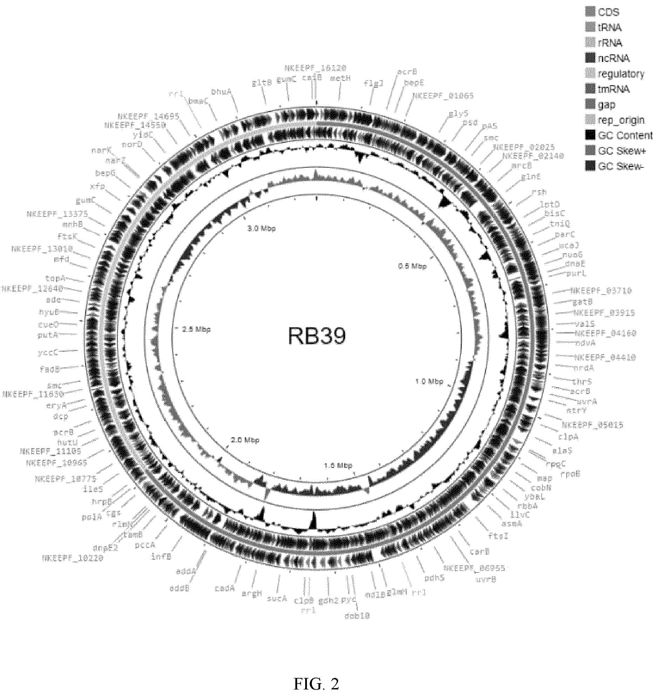

Whole genome sequencing and sequence assembly were conducted at the Institute of Animal Sciences, Chinese Academy of Agricultural Sciences, Beijing. The sequencing process was as shown in the Nanopore Library Construction Flow Diagram. After genome assembly, the results showed that the total genome length of RB39 was 3,282,094 bp, and its genome size was as shown in the circular genome map of RB39 in FIG. 2.

### Example 4 Preparation of Rough Brucella Live Vaccine (RB39 Strain)

### 4.1 Preparation of Seed Culture for Vaccine Production

The bacterial strain was streaked and transferred onto Trypticase Soy Agar (TSA) medium and cultured at 37°C for 3 days. After visual confirmation of purity, the bacterial lawn was washed off with an appropriate amount of peptone water (pH 6.5 to 7.0). The suspension was then inoculated at a 10% ratio into Tryptic Soy Broth (TSB) medium and cultured at 37°C for 2 days. After passing purity testing, the culture was stored at 2 to 8°C for no more than 30 days.

### 4.2 Preparation of Bacterial Culture for Vaccine Production

Defoamer was added to the culture medium at 0.01% (v/v). After sterilization, seed culture was inoculated at 1% (v/v) of the medium volume and fermented at 37°C for 36 hours. During cultivation, aeration was gradually increased, and 50% glucose solution was added as needed to maintain the culture pH at 7.2 ± 0.1. Each addition volume was approximately 1% (v/v) of the total medium volume. Purity testing and viable cell counting were performed after cultivation.

### 4.3 Bacterial Culture Concentration

After passing purity testing, 0.8% sodium carboxymethyl cellulose (CMC) solution was added to the culture at 1/8 (v/v) of the total medium volume. The mixture was homogenized and allowed to settle for 48 hours. The precipitated bacterial cells were then aspirated for further use, followed by purity testing and viable cell counting.

### 4.4 Vaccine Formulation and Filling

Qualified bacterial culture was uniformly mixed with a lyoprotectant containing 7% sucrose and 10% skimmed milk at a 1:7 (v/v) ratio. The mixture was quantitatively filled with a dosage of 8.0 × 10¹⁰ CFU to 1.6 × 10¹¹ CFU per cattle dose.

### 4.5 Freeze-Drying

Filled samples were immediately subjected to lyophilization according to the freeze-drying curve: loaded at 20°C; cooled at 1°C per minute to -40°C or below, held for approximately 4 hours; shelf temperature controlled at -15°C, held for 16 to 18 hours; and temperature increased by 10°C every 2 hours, held at 25°C for 2 hours before unloading. The entire process lasted approximately 36 hours.

### Example 5 Finished Product Testing of Rough Brucella Live Vaccine (RB39 Strain)

5.1 Appearance: Spongy, loose mass easily separable from the vial wall; dissolves rapidly upon adding diluent.

5.2 Purity Testing: Tested according to the current edition of the Chinese Veterinary Pharmacopoeia; results confirmed purity.

5.3 Variation Check: Diluted with peptone water to 1000 CFU/mL. Then, 100 µL of the diluted bacterial suspension was evenly spread onto TSA plates and incubated at 37°C for 3 days. Examination by crystal violet colony staining showed 100% rough-type colonies.

5.4 Viable Cell Count: The vaccine was diluted with peptone water and plated on TSA for viable cell counting. The viable count per dose was 8.0 × 10¹⁰ CFU to 1.6 × 10¹¹ CFU. Unless otherwise specified, "one dose" in subsequent examples of the present invention refers to 8.0 × 10¹⁰ CFU to 1.6 × 10¹¹ CFU.

5.5 Safety Test: The vaccine was diluted to contain 5.0×10⁹ CFU per 1.0 mL. Five mice weighing 18 to 20 g were subcutaneously injected with 0.2 mL each. All mice should survive within 6 days.

5.6 Residual Moisture Determination: Measured according to the current edition of the Chinese Veterinary Pharmacopoeia; results met specifications.

5.7 Vacuum Test: Measured according to the current edition of the Chinese Veterinary Pharmacopoeia; results met specifications.

### Example 6 Safety Testing of Rough Brucella Live Vaccine (RB39 Strain)

6.1 Mouse Safety Test: The RB39 vaccine was diluted to contain 1/20 cattle dose per 1.0 mL. Five mice weighing 18 to 22 g were subcutaneously injected with 0.25 mL each. All mice survived within 6 days.

6.2 Guinea Pig Safety Test: The RB39 vaccine was diluted with physiological saline to contain 1/80 cattle dose per 1.0 mL. Five female Hartley guinea pigs weighing 350 to 400 g were subcutaneously injected in the groin with 1 mL each. After 14 to 15 days, the guinea pigs were sacrificed, and their spleens were collected, weighed together, and emulsified. The emulsion was inoculated onto TSA medium plates. Based on the colony count, the bacterial load per gram of spleen was calculated and should not exceed 2.0 × 10⁵ CFU.

6.3 Pregnant Cattle Safety Test: The RB39 vaccine was diluted to contain one cattle dose per mL. Five pregnant adult cattle at 3 months, 5 months, and 7 months of gestation each were subcutaneously inoculated with one dose per cattle. Clinical observation was conducted for 30 days, with continuous monitoring of pregnancy and calving outcomes. No adverse reactions such as abortion occurred in the pregnant cattle, and calving proceeded normally.

6.4 Pregnant Sheep Safety Test: The RB39 vaccine was diluted to contain 1/4 dose per 1.0 mL. Ten pregnant adult goats and ten pregnant adult sheep at 2 months and 5 months of gestation each were subcutaneously inoculated with 1.0 mL per animal. Clinical observation was conducted for 30 days, with continuous monitoring of pregnancy and lambing outcomes. No adverse reactions such as abortion occurred in the pregnant sheep or goats, and lambing proceeded normally.

### Example 7 Efficacy Testing of Rough Brucella Live Vaccine (RB39 Strain)

7.1 Mouse Efficacy Test: The RB39 vaccine was diluted with physiological saline to contain 1/80 cattle dose per 1.0 mL. Ten mice weighing 18 to 22 g were subcutaneously injected in the groin with 0.1 mL of the diluted vaccine each. Thirty days later, the mice were challenged by subcutaneous injection in the groin with 100 CFU per mouse of the *Brucella melitensis* virulent strain M28 (CVCC 70003, purchased from the National Center for Veterinary Culture Collection). After 30 to 35 days of observation, the mice were sacrificed, and their spleens were subjected to bacterial culture examination. The results are shown in Table 2. As can be seen from the table, 70% (7/10) of the mice showed no virulent bacteria.

**Table 2 Mouse Efficacy Test Results**

| No. | 1 | 2 | 3 | 4 | 5 | 6 | 7 | 8 | 9 | 10 |
|---|---|---|---|---|---|---|---|---|---|---|
| Spleen Weight (g) | 0.108 | 0.082 | 0.113 | 0.102 | 0.095 | 0.098 | 0.115 | 0.134 | 0.112 | 0.086 |
| Spleen Bacterial Count (CFU) | 0 | 0 | 8.0×10² | 0 | 0 | 0 | 0 | 5.7×10⁴ | 2.0×10³ | 0 |

7.2 Guinea Pig Efficacy Test: The RB39 vaccine was diluted with physiological saline to contain 1/20 cattle dose per 1.0 mL. Ten guinea pigs weighing 350 to 400 g were subcutaneously injected in the groin with 1.0 mL of the diluted vaccine each. Thirty days later, a booster immunization was administered at the same dose and route. At 40 to 60 days after the second immunization, the guinea pigs were challenged by subcutaneous injection in the groin with 1 to 3 infectious doses (ID) (10 to 30 CFU) of the *Brucella melitensis* virulent strain M28. After 30 to 35 days of observation, the guinea pigs were sacrificed, and their spleens were subjected to bacterial culture examination. The results are shown in Table 3. As can be seen from the table, 70% (7/10) of the guinea pigs showed no virulent bacteria.

**Table 3 Guinea Pig Efficacy Test Results**

| No. | 1 | 2 | 3 | 4 | 5 | 6 | 7 | 8 | 9 | 10 |
|---|---|---|---|---|---|---|---|---|---|---|
| Spleen Weight (g) | 0.898 | 0.927 | 1.113 | 1.259 | 1.258 | 0.943 | 0.769 | 1.199 | 0.944 | 0.959 |
| Spleen Bacterial Count (CFU) | 0 | 0 | 0 | 0 | 1.5×10⁴ | 0 | 0 | 1.0×10³ | 0 | 1.0×10² |

7.3 Cattle Efficacy Test: The RB39 vaccine was diluted with physiological saline to contain one cattle dose per 1.0 mL. Five adult cattle were subcutaneously injected with one dose per cattle. Thirty days later, a booster immunization was administered at the same dose and route. At 90 days after the second immunization, the cattle were challenged by subcutaneous injection with 2.0 × 10⁷ CFU per cattle of the *Brucella melitensis* virulent strain M28. After 45 days of observation, the cattle were sacrificed, and their spleens were subjected to bacterial culture examination. Controls included an A19 vaccine group and a blank control group. The A19 control group (5 adult cattle) was subcutaneously injected with 1/60 dose per cattle according to the manufacturer's instructions. At 90 days post-immunization, they were challenged with 2.0 × 10⁷ CFU per cattle of the *Brucella melitensis* virulent strain M28. After 45 days, they were sacrificed for spleen bacterial culture. Results are shown in Table 4. The RB39 strain vaccine provided 80% protection (4/5 cattle) with no virulent bacteria detected, matching the efficacy of the A19 vaccine.

**Table 4 Cattle Efficacy Test Results**

| Group | | 1 | 2 | 3 | 4 | 5 |
|---|---|---|---|---|---|---|
| Bacterial Count per Gram of Spleen (CFU/g) | RB39 | 0 | 7.0×10² | 0 | 0 | 0 |
| | A19 | 0 | 0 | 0 | 2.0×10² | 0 |
| | Blank Control | 2.4×10³ | 1.2×10⁴ | 1.5×10³ | 3.2×10³ | 1.1×10⁴ |

7.4 Sheep Efficacy Test: The RB39 vaccine was diluted with physiological saline to contain 1/4 cattle dose per 1.0 mL. Five adult sheep and five adult goats were subcutaneously injected with 1.0 mL per animal. Thirty days later, a booster immunization was administered at the same dose and route. At 90 days after the second immunization, the animals were challenged by subcutaneous injection with 1.0×10¹⁰ CFU per animal of the *Brucella melitensis* virulent strain M28. After 45 days of observation, the animals were sacrificed, and their spleens were subjected to bacterial culture examination. Controls included an M5 vaccine group and a blank control group. The M5 control group (5 adult sheep and 5 adult goats) was subcutaneously injected with one dose per animal according to the manufacturer's instructions. At 90 days post-immunization, they were challenged with 1.0×10¹⁰ CFU per animal of the *Brucella melitensis* virulent strain M28. After 45 days, they were sacrificed for spleen bacterial culture. Results are shown in Table 5. The RB39 strain vaccine provided 80% protection (4/5 animals) with no virulent bacteria detected in both sheep and goat groups, matching the efficacy of the M5 vaccine.

**Table 5 Sheep Efficacy Test Results**

| Group | | 1 | 2 | 3 | 4 | 5 |
|---|---|---|---|---|---|---|
| Bacterial Count per Gram of Spleen (CFU/g) - Sheep Group | RB39 | 0 | 0 | 9.0×10² | 0 | 0 |
| | M5 | 0 | 0 | 0 | 0 | 3.2×10³ |
| | Blank Control | 3.8×10⁴ | 1.7×10³ | 2.5×10⁴ | 1.8×10⁴ | 1.2×10⁵ |
| Bacterial Count per Gram of Spleen (CFU/g) - Goat Group | RB39 | 3.0×10² | 0 | 0 | 0 | 0 |
| | M5 | 0 | 1.2×10² | 0 | 0 | 0 |
| | Blank Control | 2.6×10³ | 1.4×10³ | 3.6×10³ | 1.6×10⁴ | 2.1×10³ |

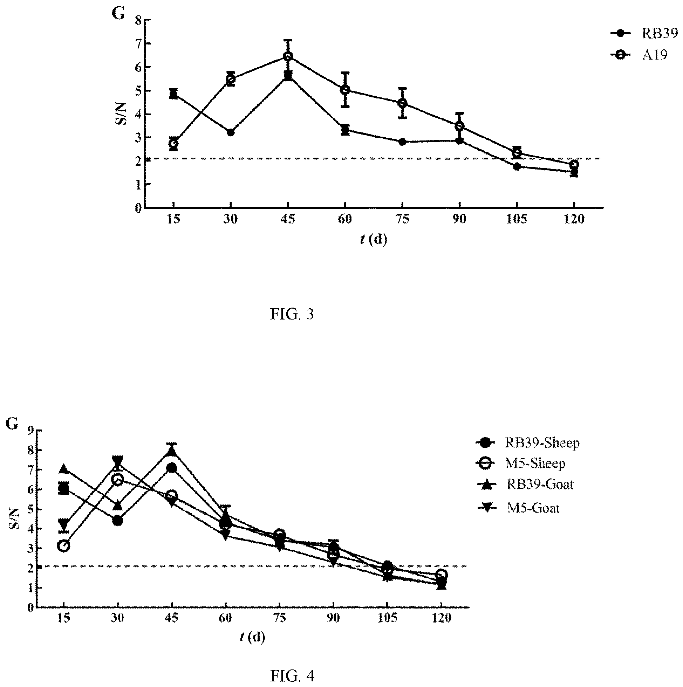

7.5 Antibody Level Monitoring in Immunized Cattle and Sheep: For the cattle and sheep in efficacy tests 7.3 and 7.4, blood samples were collected at 15-day intervals from the first immunization until challenge. Sera were separated simultaneously from control groups. Inactivated RB39, A19, and M5 bacterial suspensions (1.0×10¹⁰ CFU/mL) were sonicated at 350W for 40 minutes and centrifuged. The supernatant containing lysed bacterial proteins was diluted with PBS buffer to 10 µg/mL protein concentration for coating ELISA plates (100 µL per well), followed by incubation at 2 to 8°C for 16 hours. After one wash with PBST, wells were blocked with 2% bovine serum albumin at 2 to 8°C for 24 hours. Following three PBST washes, plates were ready for use. Sera from different immunization groups were added to corresponding antigen-coated plates. Blank control group sera were added to all antigen-coated plates as negative controls. Reaction proceeded at 37°C for 30 minutes. HRP-polyclonal rabbit anti-bovine IgG or HRP-polyclonal mouse anti-sheep IgG working solution (100 µL/well) was added and incubated at 37°C for 30 minutes. After three PBST washes, 100 µL substrate chromogen solution was added per well and developed protected from light for 15 minutes. The reaction was terminated with 50 µL stop solution per well. Optical density (OD) values were read at 450 nm using a microplate reader. Sample S/N values (Od₄₅₀ₙₘ of immunized sample / Od₄₅₀ₙₘ of negative control sample) were calculated. Results are shown in FIG. 3 and FIG. 4. Results indicated that: After primary immunization with RB39 vaccine, antibody levels in cattle and sheep peaked at day 15, then declined by day 30. The highest antibody levels occurred at day 15 post-booster (45 days after primary immunization), followed by a gradual decline. A19 and M5 vaccines induced peak antibody levels at 30 to 45 days post-immunization, with a declining trend thereafter, consistent with RB39. Antibody titers showed no significant difference between RB39 and A19/M5 strains (P > 0.05).

### Example 8 Preparation of Rough Brucella RB39 Strain Rose Bengal Plate Agglutination Test Antigen

8.1 Preparation of Rough *Brucella* RB39 Strain Positive Serum: The rough *Brucella* RB39 strain was inoculated onto TSA medium and cultured at 37°C for 3 days. The culture was harvested and prepared into a bacterial suspension of 4×10¹⁰ CFU/mL, followed by inactivation in an 80°C water bath for 2 hours. The inactivated antigen was used to immunize *Brucella* antibody-negative healthy cattle via subcutaneous injection of 4 mL per cattle in the neck region. One month later, a booster immunization was administered at double the dose. Two weeks after the booster, blood was collected, and tube agglutination tests were performed using the corresponding antigen. The agglutination titer was determined to be no less than 1:100. Venous blood was collected, and serum was separated. After sterile filtration through a 0.22 µm filter, the positive serum was diluted with commercial fetal bovine serum (Shuangru Biotechnology, Cat. No. S711-001S) to a tube agglutination titer of 1:100 (equivalent to 100 IU/mL). ProClin 300 was added to a final concentration of 0.1%, followed by aseptic aliquoting and storage below -15°C.

8.2 Preparation of Rough *Brucella* RB39 Strain Rose Bengal Stained Antigen: The rough *Brucella* RB39 strain was inoculated onto TSA medium and cultured at 37°C for 3 days. The culture was harvested and prepared into a concentrated suspension with an Od₆₀₀ₙₘ of 1.5, followed by inactivation in an 80°C water bath for 2 hours. A volume ratio of 0.5% of 4% Rose Bengal dye was added to the inactivated bacterial suspension. The mixture was thoroughly stirred for 30 minutes and centrifuged at 8,000 rpm for 20 minutes. The supernatant was discarded. The pellet was resuspended in Tris buffer at a ratio of 4 mL per gram of pellet weight. After thorough mixing for 30 minutes, the suspension was standardized.

8.3 Calibration of Rough *Brucella* RB39 Strain Rose Bengal Plate Agglutination Test Antigen: The Rose Bengal-stained antigen was diluted in Tris buffer to 1:3, 1:4, 1:5, and 1:6 dilutions. The rough *Brucella* RB39 strain positive serum was diluted with Tris buffer to 10 IU/mL and 5 IU/mL. Then, 0.03 mL of each diluted serum was mixed with an equal volume of each diluted stained antigen for plate agglutination reactions, with results observed within 4 minutes. Negative serum and smooth *Brucella*-positive serum were simultaneously included as controls The antigen dilution at which the rough *Brucella* RB39 strain positive serum at 10 IU/mL showed a "+" reaction and at 5 IU/mL showed a "-" reaction was determined as the working concentration. Results are shown in Table 6. Based on Table 6 results, the stained antigen was diluted to the working concentration (1:4) with Tris buffer to obtain the final rough *Brucella* RB39 strain Rose Bengal plate agglutination test antigen.

**Table 6 Calibration Results of Rough Brucella RB39 Strain Rose Bengal Plate Agglutination Test Antigen**

| Dilution of Rough Rose Bengal Antigen | Rough *Brucella* RB39 Strain Positive Serum | | Control Sera | |
|---|---|---|---|---|
| | 10IU/mL | 5IU/mL | Negative Serum | Smooth *Brucella*-Positive Serum |
| 1:3 | - | - | - | - |
| 1:4 | + | - | - | - |
| 1:5 | + | + | - | - |
| 1:6 | ++ | + | - | - |

| | | | | |
|---|---|---|---|---|
| Note: ++: Definitive aggregates, slightly clear fluid; +: Partial aggregates, turbid fluid; -: No aggregates, uniformly turbid fluid. | | | | |

### Example 9 Differential Diagnosis Testing after Immunization with Rough Brucella Live Vaccine (RB39 Strain)

9.1 Immunization: The RB39 vaccine was diluted with physiological saline to contain one cattle dose per 1.0 mL. Five adult cattle were subcutaneously injected with one dose as the immunized group; a non-immunized control group (5 cattle) was simultaneously established. The RB39 vaccine was diluted with physiological saline to contain 1/4 dose per 1.0 mL. Five adult sheep and five adult goats were subcutaneously injected with 1.0 mL per animal as immunized groups; non-immunized control groups (5 animals per species) were established.

9.2 Blood Collection: Blood was collected from all immunized and control group animals 14 days post-immunization, and sera were separated. Additionally, serum samples were collected from cattle, goats, and sheep in farms without prior brucellosis vaccination. Five serum samples positive by both Rose Bengal plate agglutination test (smooth type) and complement fixation test were selected as clinically infected positive sera.

9.3 Differential Diagnosis Testing: Sera from RB39-vaccinated animals, non-immunized controls, and clinically infected positive sera were tested using: Rough *Brucella* RB39 strain Rose Bengal plate agglutination test antigen and Smooth-type Rose Bengal plate agglutination test antigen. Results are shown in Table 7. Sera from cattle, goats, and sheep immunized with the rough *Brucella* live vaccine (RB39 strain) showed positive reactions (+) with the rough *Brucella* RB39 strain Rose Bengal plate agglutination test antigen and no reaction (-) with the smooth-type Rose Bengal plate agglutination test antigen. Sera from clinically infected cattle, goats, and sheep showed no reaction (-) with the rough *Brucella* RB39 strain Rose Bengal plate agglutination test antigen and positive reactions (+) with the smooth-type Rose Bengal plate agglutination test antigen. Sera from the non-immunized control group showed no reaction (-) with both antigens. Therefore, antibodies induced by clinical RB39 vaccination and those from clinical infection could be differentiated, enabling differential diagnosis of *Brucella* immunization versus infection.

**Table 7 Differential Diagnosis Results by Rose Bengal Plate Agglutination Test**

| Group | Sample No. | Rough Brucella RB39 Strain Rose Bengal Plate Agglutination Test Antigen | Smooth-Type Rose Bengal Plate Agglutination Test Antigen |
|---|---|---|---|
| RB39-Vaccinated Cattle | N1 | + | - |
| | N2 | + | - |
| | N3 | + | - |
| | N4 | + | - |
| | N5 | + | - |
| Non-Immunized Cattle Control | N6 | - | - |
| | N7 | - | - |
| | N8 | - | - |
| | N9 | - | - |
| | N10 | - | - |
| Clinically Infected Cattle | N11 | - | + |
| | N12 | - | + |
| | N13 | - | + |
| | N14 | - | + |
| | N15 | - | + |
| RB39-Vaccinated Sheep | M1 | + | - |
| | M2 | + | - |
| | M3 | + | - |
| | M4 | + | - |
| | M5 | + | - |
| Non-Immunized Sheep Control | M6 | - | - |
| | M7 | - | - |
| | M8 | - | - |
| | M9 | - | - |
| | M10 | - | - |
| Clinically Infected Sheep | M11 | - | + |
| | M12 | - | + |
| | M13 | - | + |
| | M14 | - | + |
| | M15 | - | + |
| RB39-Vaccinated Goats | S1 | + | - |
| | S2 | + | - |
| | S3 | + | - |
| | S4 | + | - |
| | S5 | + | - |
| Non-Immunized Goat Control | S6 | - | - |
| | S7 | - | - |
| | S8 | - | - |
| | S9 | - | - |
| | S10 | - | - |
| Clinically Infected | S11 | - | + |
| Goats | S12 | - | + |
| | S13 | - | + |
| | S14 | - | + |
| | S15 | - | + |

| | | | |
|---|---|---|---|
| Note: +: Positive (Agglutination observed); -: Negative (No reaction). | | | |

The above described is preferred embodiments of the present invention. It should be noted that for those skilled in the art, a plurality of improvements and modifications may be made without departing from the principles of the present invention, which should also be considered as the scope of protection of the present invention.

## Claims

1. A rough attenuated strain RB39 of *Brucella,* deposited on February 17, 2025 at the China General Microbiological Culture Collection Center (CGMCC) under accession number CGMCC 46377.

2. The rough attenuated strain RB39 of *Brucella* according to claim 1, **characterized in that** colonies thereof are identified as 100% rough phenotype by crystal violet staining assay and identified as *Brucella abortus* by AMOS-PCR assay.

3. Use of the rough attenuated strain RB39 of *Brucella* according to claim 1 or 2 in a preparation of a brucellosis vaccine.

4. A brucellosis vaccine, comprising a rough attenuated strain RB39 of *Brucella* deposited on February 17, 2025 at the China General Microbiological Culture Collection Center (CGMCC) under accession number CGMCC 46377.

5. The brucellosis vaccine according to claim 4, further comprising a lyoprotectant.

6. Use of the rough attenuated strain RB39 of *Brucella* according to claim 1 or 2 in a preparation of an antigen for detection of *Brucella* antibodies produced by an animal immunized with the brucellosis vaccine according to claim 4 or 5,
wherein the detection allows differentiation between vaccination and natural infection.

7. The use according to claim 6, **characterized in that** the detection employs the RBT method, the SAT method, the CFT method, or the ELISA method.

8. An antigen for detection of *Brucella* antibodies produced by an animal immunized with the brucellosis vaccine according to claim 4 or 5, wherein the *Brucella* antibodies are obtained by culturing and inactivating the rough attenuated strain RB39 of *Brucella* according to claim 1 or 2.

9. The antigen according to claim 8, **characterized in that** the antigen is for detection of *Brucella* antibodies by the RBT method, and a preparation method thereof comprising:
preparing a bacterial suspension from the rough attenuated strain RB39 of *Brucella* according to claim 1 or 2 using physiological saline, followed by inactivation;
adding Rose Bengal dye to the bacterial suspension after inactivation at a volume ratio of 0.5:100 for staining to obtain stained bacterial cells; and
resuspending the stained bacterial cells in Tris buffer to a predetermined concentration.

10. A kit for detecting *Brucella* antibodies, comprising the antigen according to claim 8 or 9.
